# FASCICULE DE BREVET EUROPEEN

(11) **EP 0 755 907 B1**
(45) Date de publication et mention de la délivrance du brevet: **01.12.1999**
(21) Numéro de dépôt: 96401561.4
(22) Date de dépôt: 15.07.1996
(51) Int. Cl.: C07C 7/05

(54) **Procédé de séparation d'alpha-oléfines par distillation d'un effluent comprenant de l'éthylène et du butène-1**
Verfahren zur Abtrennung durch Distillierung von Ethylen aus Mischungen von Ethylen und Buten-1
Process for the separation of alpha-olefins by destillation from an effluent comprising ethylene and butene-1

(30) Priorité: 24.07.1995 FR 9509059
(43) Date de publication de la demande: 29.01.1997
(73) Titulaire: INSTITUT FRANCAIS DU PETROLE, 92502 Rueil-Malmaison (FR)
(72) Inventeur: Boucot, Pierre, 69360 Ternay (FR); Chodorge, Jean-Alain, 92160 Antony (FR); Forestiere, Alain, 69390 Vernaison (FR); Glaize, Yves, 69360 Saint Symphorien d'Ozon (FR); Hugues, François, Charly 69390 Vernaison (FR)

(56) Documents cités:
- US-A- 2 775 103

## Description

La présente invention a pour objet un procédé industriel amélioré de séparation d'un mélange comprenant de l'éthylène, du butène-1, des alpha-oléfines comprenant au moins 6 atomes de carbone par molécule et éventuellement des hydrocarbonés plus lourds, la teneur dudit mélange en éthylène étant comprise entre 30 et 70% en poids. Selon une mise en oeuvre préférée du procédé de la présente invention, ledit mélange est issu d'une zone d'oligomérisation homogène en phase liquide de l'éthylène et comprend de l'éthylène n'ayant pas réagi, du butène-1, de l'hexène-1, de l'octène-1, des oléfines comprenant au moins 10 atomes de carbone par molécule, du catalyseur inhibé, et le solvant de l'oligomérisation.

L'oligomérisation de l'éthylène est le plus souvent effectuée dans un procédé de catalyse en phase liquide, généralement homogène, en présence ou non de solvant, par un catalyseur de type Ziegler qui comprend généralement un composé d'un métal tel que le titane, le chrome, le zirconium et un composé organoaluminique, à une température comprise entre 100 et 200°C et à une pression comprise entre 0,5 et 20 MPa. Selon une mise en oeuvre préférée du procédé de la présente invention, ledit procédé de séparation suit un procédé d'oligomérisation en présence de solvant.

L'effluent issu de l'oligomérisation de l'éthylène comprend de l'éthylène non converti et des alpha-oléfines, principalement du butène-1, de l'hexène-1, de l'octène-1, ainsi que le solvant de l'oligomérisation et les alpha-oléfines C₁₀+ (c'est-à-dire comportant au moins 10 atomes de carbone par molécule). Ledit effluent comprend aussi du catalyseur. A la sortie de l'oligomérisation, le catalyseur est désactivé par tout composé connu de l'homme du métier pour son activité inhibitrice du catalyseur d'oligomérisation. Ledit composé est généralement une amine, et de préférence une amine à longue chaîne, c'est-à-dire comportant au moins six atomes de carbone par molécule. Ainsi la charge à séparer comprend de préférence essentiellement de l'éthylène non converti, du butène-1, de l'hexène-1, de l'octène-1, le solvant de l'oligomérisation, le composé résultant de la désactivation du catalyseur par l'inhibiteur, que l'on appelle ici pour simplifier catalyseur inhibé, et les alpha-oléfines C₁₀+.

La séparation de l'éthylène des alpha-oléfines telles que le butène-1, l'hexène-1 et l'octène-1, du solvant, du catalyseur inhibé et des alpha-oléfines C₁₀+, pose un problème particulier lié d'une part à la forte proportion d'éthylène et d'autre part à la sensibilité en température des alpha-oléfines qui impose une température en fond de zone de distillation inférieure à 250°C et de préférence inférieure à 200°C. Ceci donne des températures de tête de colonne de distillation réalisables industriellement mais avec de forts coûts opératoires. En effet, elles sont par exemple le plus souvent inférieures à -30°C dans le cas où la zone de distillation consiste en une colonne à distiller telle que la température de fond de colonne est de 200°C, où le solvant est l'orthoxylène et où l'on veut séparer en tête principalement la majeure partie de l'éthylène contenu dans la charge.

L'objet de l'invention est d'apporter une solution au problème précédent, où l'on doit séparer par distillation, dans une zone de distillation qui est généralement une colonne à distiller, un mélange tel que décrit précédemment. La solution objet de l'invention consiste à ajouter volontairement du butène-1 supplémentaire au mélange que l'on veut séparer qui est de préférence un mélange issu de l'oligomérisation de l'éthylène, que ce soit avant la séparation par distillation, dans le mélange, ou bien directement dans ladite colonne, de préférence sur les plateaux supérieurs, ou encore en partie dans le mélange et en autre partie directement dans la colonne. Ainsi la température en fond de ladite colonne est maintenue à la température de stabilité des alpha-oléfines, et l'on peut séparer en tête de colonne un effluent comprenant principalement la majeure partie de l'éthylène compris dans ledit mélange et éventuellement une partie du butène-1 compris dans ledit mélange, et en fond de colonne un mélange comprenant tout ou une autre partie du butène-1, les alpha-oléfines comprenant plus de 6 atomes de carbone par molécule, le solvant hydrocarboné et le catalyseur inhibé. La solution précédemment exposée est généralisable à tout cas où l'on doit séparer par distillation, dans une zone de distillation qui est généralement une colonne à distiller, un mélange du même type.

L'intérêt de notre solution est l'obtention d'une température de condensation en tête de colonne raisonnable. Les effluents obtenus peuvent être pompés dans une colonne à plus haute pression comprise dans le train de séparation qui est en aval de la zone de distillation selon l'invention. On évite ainsi l'emploi de compresseurs qui sont plus délicats que les pompes du point de vue maintenance.

Ainsi, le procédé selon l'invention est un procédé de séparation d'un mélange comprenant de l'éthylène, une coupe hydrocarbonée C₄, c'est-à-dire comprenant principalement des hydrocarbures comportant 4 atomes de carbone par molécule, comprenant en majeure partie du butène-1, une coupe hydrocarbonée C₆, c'est-à-dire comprenant principalement des hydrocarbures comportant 6 atomes de carbone par molécule, comprenant en majeure partie des alpha-oléfines comprenant au moins 6 atomes de carbone par molécule et éventuellement des produits hydrocarbonés comportant au moins 7 atomes de carbone par molécule, la teneur dudit mélange en éthylène étant comprise entre 30 et 70% en poids, de préférence entre 40 et 60% en poids, dans lequel on effectue la séparation dans une zone de distillation, ce qui permet d'obtenir une fraction de tête de distillation comprenant la majeure partie de l'éthylène présent dans ledit mélange et entre 0 et 100% en poids, de préférence entre 50 et 70% en poids, et de manière préférée entre 55 et 65% en poids du butène-1 présent dans ledit mélange, ledit procédé étant caractérisé en ce que l'on alimente aussi ladite zone en butène-1 supplémentaire, en quantité comprise entre 1 et 40 fois la quantité (en poids) de butène-1 présent dans ledit mélange, de préférence entre 2 et 25 fois ladite quantité, et de manière encore plus préférée entre 5 et 20 fois ladite quantité.

Ainsi la température de condensation en tête de la zone de distillation en vue du reflux est généralement comprise entre -35 et +60°C, de préférence entre +20 et +50°C.

Le procédé selon l'invention comporte au moins deux mises en oeuvre possibles. Une première mise en oeuvre du procédé selon l'invention comprend l'ajout audit mélange avant son entrée dans la zone de distillation du butène-1 supplémentaire, l'ensemble [mélange + butène-1 supplémentaire] alimentant ainsi ladite zone. Une autre mise en oeuvre du procédé selon l'invention est telle que le butène-1 supplémentaire est injecté directement dans la zone de distillation indépendamment de l'alimentation en ledit mélange, de préférence sur les plateaux supérieurs de ladite zone. Mais il est aussi envisageable dans le cadre de la présente invention de mettre en oeuvre une troisième possibilité qui consiste à combiner les deux possibilités précédentes, c'est-à-dire qu'une partie du butène-1 est ajoutée au mélange et que l'autre partie du butène-1 est injectée directement dans la zone de distillation.

Une réalisation préférée du procédé selon l'invention est telle que ledit mélange est issu d'une zone d'oligomérisation homogène en phase liquide de l'éthylène et comprend de l'éthylène n'ayant pas réagi, du butène-1, de l'hexène-1, de l'octène-1, des oléfines comprenant au moins 10 atomes de carbone par molécule et le solvant de l'oligomérisation. De préférence, ledit mélange comprend en outre le catalyseur de l'oligomérisation qui a été désactivé par un inhibiteur, c'est-à-dire le catalyseur inhibé. L'inhibiteur est généralement choisi dans le groupe formé par les amines, de préférence, il est choisi dans le groupe formé par les amines à longue chaîne, c'est-à-dire les amines qui comportent au moins 6 atomes de carbone par molécule.

Le solvant est généralement choisi parmi les composés connus de l'homme du métier pour leur activité de solvant de l'oligomérisation de l'éthylène. Mais tout autre solvant connu de l'homme du métier pour avoir un (des) point(s) d'ébullition semblable(s) et des propriétés comparables est envisageable. Le solvant est généralement hydrocarboné, mais un solvant non hydrocarboné est aussi envisageable. Le solvant est de préférence choisi parmi les composés hydrocarbonés comprenant au moins un noyau aromatique. De manière plus préférée, ledit solvant est constitué principalement d'un mélange de xylènes et de manière encore plus préférée ledit solvant est l'orthoxylène.

Dans le cadre de ladite réalisation préférée du procédé selon l'invention, ledit procédé comporte en aval de la zone de distillation un train de séparation, alimenté au moins par la majeure partie du produit de fond de ladite zone. Ledit train de séparation peut être aussi alimenté séparément par le produit de tête de ladite zone, surtout lorsque ledit produit de tête comprend du butène-1. La zone de séparation ainsi constituée dudit train de séparation et de ladite zone de distillation peut réaliser différentes séparations ultérieures. Ainsi, dans un premier mode de réalisation, elle permet d'obtenir un effluent comportant en majeure partie l'éthylène, un effluent comportant en majeure partie le butène-1, un effluent comportant en majeure partie l'hexène-1 et l'octène-1, et un effluent comportant en majeure partie les alpha-oléfines comportant au moins 10 atomes de carbone par molécule, le catalyseur inhibé et le solvant. Dans un deuxième mode de réalisation, elle permet d'obtenir un effluent comportant en majeure partie l'éthylène, un effluent comportant en majeure partie le butène-1, un effluent comportant en majeure partie l'hexène-1, un effluent comportant en majeure partie l'octène-1, et un effluent comportant en majeure partie les alpha-oléfines comportant au moins 10 atomes de carbone par molécule, le catalyseur inhibé et le solvant. Enfin, dans un troisième mode de réalisation, elle permet d'obtenir un effluent comportant en majeure partie l'éthylène, un effluent comportant en majeure partie le butène-1, un effluent comportant en majeure partie l'hexène-1, un effluent comportant en majeure partie l'octène-1, un effluent comportant en majeure partie le solvant et un effluent comportant en majeure partie les alpha-oléfines comportant au moins 10 atomes de carbone par molécule et le catalyseur inhibé.

Dans le cadre de ladite réalisation préférée du procédé selon l'invention et selon l'un quelconque des trois modes de réalisation décrits précedemment, ledit procédé permet ainsi d'obtenir au moins un effluent comportant en majeure partie de l'éthylène, et de préférence le procédé est tel qu'une partie de l'effluent comportant en majeure partie l'éthylène est recyclée en majeure partie en zone d'oligomérisation. Ladite partie peut comporter en plus de l'éthylène de 0 à 20% en poids de butène-1, de préférence de 3 à 10% en poids de butène-1, c'est-à-dire qu'il peut ne pas comporter du tout de butène-1 ou qu'il peut comporter du butène-1, à mesure de 0 à 20% en poids, de préférence de 3 à 10% en poids.

L'exemple qui suit est non limitatif et il sert à illustrer l'invention.

### EXEMPLE

On envisage de séparer un mélange issu de l'oligomérisation comportant de l'éthylène (771 kg/h) et des alpha-oléfines telles que le butène-1 (273 kg/h), l'hexène-1 (206 kg/h), l'octène-1 (112 kg/h), ainsi que les oligomères comportant au moins dix atomes de carbone par molécule (167 kg/h), le solvant de l'oligomérisation qui est dans ce cas de l'orthoxylène (3712 kg/h) et le catalyseur désactivé par addition de dodécylamine. La zone de distillation consiste en une colonne à distiller et l'on veut que la température de fond de colonne soit de 200°C.

Si l'on veut séparer en tête pratiquement tout l'éthylène contenu dans la charge, cela impose une pression de 10,5 bars (soit 1,05 MPa) et une température de condensation en vue de reflux de -50,3°C. Si l'on veut séparer en tête de colonne le mélange d'-oléfines comportant entre 2 et 8 atomes de carbone par molécule, cela impose une pression de 3,5 bars (soit 0,35 MPa) et une température de condensation en vue de reflux de -73°C. Les deux températures de condensation en vue de reflux ainsi calculées sont industriellement peu envisageables économiquement.

De plus, on veut obtenir une température de tête de colonne de 40°C, ce qui est compatible avec l'utilisation d'un condenseur à eau. Alors, la solution selon l'invention consiste à introduire dans le mélange issu de l'oligomérisation 12 fois la quantité de butène-1 contenu dans ledit mélange, la pression optimale de la colonne étant de 29 bars (2,9 MPa) et le rendement molaire de butène-1 dans la fraction de tête étant de l'ordre de 50%.

## Revendications

1. Procédé de séparation d'un mélange comprenant de l'éthylène, une coupe hydrocarbonée comprenant principalement des hydrocarbures comportant 4 atomes de carbone par molécule, comprenant en majeure partie du butène-1, une coupe hydrocarbonée comprenant principalement des hydrocarbures comportant 6 atomes de carbone par molécule, comprenant en majeure partie des alpha-oléfines comprenant au moins 6 atomes de carbone par molécule, la teneur dudit mélange en éthylène étant comprise entre 30 et 70% en poids, dans lequel on effectue la séparation dans une zone de distillation, ce qui permet d'obtenir une fraction de tête de distillation comprenant la majeure partie de l'éthylène présent dans ledit mélange et entre 0 et 100% en poids du butène-1 présent dans ledit mélange, ledit procédé étant caractérisé en ce que l'on alimente aussi ladite zone en butène-1 supplémentaire, en quantité comprise entre 1 et 40 fois la quantité (en poids) de butène-1 présent dans ledit mélange.

2. Procédé selon la revendication 1 tel que le mélange comprend en outre des produits hydrocarbonés comportant au moins 7 atomes de carbone par molécule.

3. Procédé selon l'une des revendications 1 ou 2 tel que le butène-1 supplémentaire est ajouté audit mélange avant son entrée dans la zone de distillation, et ainsi l'ensemble [mélange + butène-1 supplémentaire] alimente ladite zone.

4. Procédé selon l'une des revendications 1 ou 2 tel que le butène-1 supplémentaire est injecté directement dans la zone de distillation indépendamment de l'alimentation en ledit mélange.

5. Procédé selon l'une des revendications 1 ou 2 tel qu'une partie du butène-1 est ajoutée au mélange et que l'autre partie du butène-1 est injectée directement dans la zone de distillation indépendamment de l'alimentation en ledit mélange.

6. Procédé selon l'une des revendications 1 à 5 tel que ledit mélange est issu d'une zone d'oligomérisation homogène en phase liquide de l'éthylène et comprend de l'éthylène n'ayant pas réagi, du butène-1, de l'hexène-1, de l'octène-1, des alpha-oléfines comprenant au moins 10 atomes de carbone par molécule, le catalyseur de l'oligomérisation qui a été désactivé par un inhibiteur ou catalyseur inhibé et le solvant de l'oligomérisation.

7. Procédé selon la revendication 6 comportant suite à la zone de distillation un train de séparation, alimenté au moins par la majeure partie du produit de fond de ladite zone, la zone de séparation ainsi constituée dudit train de séparation et de ladite zone de distillation permettant d'obtenir un effluent comportant en majeure partie l'éthylène, un effluent comportant en majeure partie le butène-1, un effluent comportant en majeure partie l'hexène-1 et l'octène-1, et un effluent comportant en majeure partie les alpha-oléfines comportant au moins 10 atomes de carbone par molécule, le catalyseur inhibé et le solvant.

8. Procédé selon la revendication 6 comportant suite à la zone de distillation un train de séparation, alimenté au moins par la majeure partie du produit de fond de ladite zone, la zone de séparation ainsi constituée dudit train de séparation et de ladite zone de distillation permettant d'obtenir un effluent comportant en majeure partie l'éthylène, un effluent comportant en majeure partie le butène-1, un effluent comportant en majeure partie l'hexène-1, un effluent comportant en majeure partie l'octène-1, et un effluent comportant en majeure partie les composés comportant au moins 10 atomes de carbone par molécule, le catalyseur inhibé et le solvant.

9. Procédé selon la revendication 6 comportant suite à la zone de distillation un train de séparation, alimenté au moins par la majeure partie du produit de fond de ladite zone, la zone de séparation ainsi constituée dudit train de séparation et de ladite zone de distillation permettant d'obtenir un effluent comportant en majeure partie l'éthylène, un effluent comportant en majeure partie le butène-1, un effluent comportant en majeure partie l'hexane-1, un effluent comportant en majeure partie l'octène-1, un effluent comportant en majeure partie le solvant et un effluent comportant en majeure partie les alpha-oléfines comportant au moins 10 atomes de carbone par molécule et le catalyseur inhibé.

10. Procédé selon l'une des revendications 6 à 9 tel qu'une partie de l'effluent comportant en majeure partie l'éthylène est recyclée en majeure partie en zone d'oligomérisation.

11. Procédé selon la revendication 10 tel que ladite partie comporte en plus de l'éthylène de 0 à 20% en poids de butène-1.

## Claims

1. A process for the separation of a mixture comprising ethylene, a hydrocarbon cut principally comprising hydrocarbons containing 4 carbon atoms per molecule, the major portion being comprised by 1-butene, a hydrocarbon cut principally comprising hydrocarbons containing 6 carbon atoms per molecule, the major portion of which being alpha-olefins containing at least 6 carbon atoms per molecule, the ethylene content of said mixture being in the range 30% to 70% by weight, in which separation is effected in a distillation zone to obtain an overhead fraction comprising the major portion of the ethylene present in said mixture and between 0% and 100% by weight of the 1-butene present in said mixture, the process being characterized in that said zone is also supplied with supplemental 1-butene in an amount in the range 1 to 40 times the quantity (by weight) of 1-butene present in said mixture.

2. A process according to claim 1, in which the mixture further comprises hydrocarbon products containing at least 7 carbon atoms per molecule.

3. A process according to claim 1 or claim 2, in which the supplemental 1-butene is added to said mixture before its entry into the distillation zone, so that the [mixture + supplemental 1-butene] ensemble is supplied to said zone.

4. A process according to claim 1 or claim 2, in which the supplemental 1-butene is injected directly into the distillation zone independently of the supply of said mixture.

5. A process according to claim 1 or claim 2, in which a portion of the 1-butene is added to the mixture and the other portion of the 1-butene is injected directly into the distillation zone independently of the supply of said mixture.

6. A process according to any one of claims 1 to 5, in which the mixture originates from a homogeneous liquid ethylene oligomerisation zone and comprises unreacted ethylene, 1-butene, 1-hexene, 1-octene, alpha-olefins containing at least 10 carbon atoms per molecule, the oligomerisation catalyst which has been deactivated by an inhibitor, i.e., inhibited catalyst, and the oligomerisation solvent.

7. A process according to claim 6 comprising a separation train downstream of the distillation zone, said separation train being supplied with the major portion of the product from the bottom of said zone, the separation zone thus constituted by said separation train and said distillation zone producing an effluent comprising ethylene as its major portion, an effluent comprising 1-butene as its major portion, an effluent comprising 1-hexene and 1-octene as its major portion, and an effluent comprising alpha-olefins containing at least 10 carbon atoms per molecule as its major portion, along with inhibited catalyst and solvent.

8. A process according to claim 6 comprising a separation train downstream of the distillation zone, said separation train being supplied with the major portion of the product from the bottom of said zone, the separation zone thus constituted by said separation train and said distillation zone being capable of producing an effluent comprising ethylene as its major portion, an effluent comprising 1-butene as its major portion, an effluent comprising 1-hexene as its major portion, an effluent comprising 1-octene as its major portion, and an effluent comprising compounds containing at least 10 carbon atoms per molecule as its major portion, along with inhibited catalyst and solvent.

9. A process according to claim 6 comprising a separation train downstream of the distillation zone, said separation train being supplied with the major portion of the product from the bottom of said zone, the separation zone thus constituted by said separation train and said distillation zone producing an effluent comprising ethylene as its major portion, an effluent comprising 1-butene as its major portion, an effluent comprising 1-hexene as its major portion, an effluent comprising 1-octene as its major portion, an effluent comprising the solvent as its major portion, and an effluent comprising alpha-olefins containing at least 10 carbon atoms per molecule as its major portion, along with inhibited catalyst.

10. A process according to any one of claims 6 to 9, in which a major portion of the portion of the effluent comprising ethylene as its major portion is recycled to the oligomerisation zone.

11. A process according to claim 10, in which said portion further comprises 0% to 20% by weight of 1-butene in addition to the ethylene.

## Patentansprüche

1. Verfahren zum Trennen eines Gemisches, umfassend: Ethylen, eine kohlenwasserstoffhaltige Fraktion, die hauptsächlich Kohlenwasserstoffe mit 4 Kohlenstoffatomen pro Molekül umfaßt, und zum größeren Teil Buten-1 umfaßt, eine kohlenwasserstoffhaltige Fraktion, die hauptsächlich Kohlenwasserstoffe mit 6 Kohlenstoffatomen pro Molekül umfaßt und zum größeren Teil Alpha-Olefine aufweist, die wenigstens 6 Kohlenstoffatome pro Molekül umfassen, wobei der Gehalt dieses Gemisches an Ethylen zwischen 30 und 70 Gew. -% liegt, bei dem man die Trennung in einer Destillationszone durchführt, was es ermöglicht, eine Destillationskopffraktion zu erhalten, die den größeren Anteil an in diesem Gemisch enthaltenem Ethylen und zwischen 0 und 100 Gew. -% von in dieser Charge vorhandenem Buten-1 umfaßt, wobei das Verfahren sich dadurch auszeichnet, daß man auch diese Zone mit zusätzlichem Buten-1 in einer Menge zwischen dem 1 und 40-fachen der Menge (in Gewicht) des in diesem Gemisch vorhandenen Buten-1 speist.

2. Verfahren nach Anspruch 1, derart, daß das Gemisch im übrigen kohlenwasserstoffhaltige Produkte umfaßt, die wenigstens 7 Atome Kohlenstoff pro Molekül umfassen.

3. Verfahren nach einem der Ansprüche 1 oder 2, derart, daß das zusätzliche Buten-1 dem Gemisch vor seinem Eintritt in die Destillationszone zugegeben wird und daß auch die Gesamtheit [Gemisch + zusätzliches Buten-1] diese Zone speist.

4. Verfahren nach einem der Ansprüche 1 oder 2, derart, daß das zusätzliche Buten-1 direkt in die Destillationszone unabhängig von der Speisung mit diesem Gemisch injiziert wird.

5. Verfahren nach einem der Ansprüche 1 oder 2, derart, daß ein Teil des Buten-1 dem Gemisch zugegeben wird und daß der andere Teil des Buten-1 direkt in die Destillationszone, unabhängig von der Speisung mit diesem Gemisch, injiziert wird.

6. Verfahren nach einem der Ansprüche 1 bis 4, derart, daß dieses Gemisch aus einer in flüssiger Phase des Ethylens homogenen Oligomerisierungszone stammt und umfaßt: Ethylen, das nicht reagiert hat, Buten-1, Hexen-1, Octen-1, Alpha-Olefine, die wenigstens 10 Kohlenstoffatome pro Molekül umfassen, den Oligomerisierungskatalysator, der durch einen Inhibitor oder inhibierten Katalysator desaktiviert wurde sowie das Oligomerisierungslösungsmittel.

7. Verfahren nach Anspruch 6, anschließend an die Destillationszone eine Separatorkette umfassend, die wenigstens zum größeren Teil mit dem Bodenprodukt dieser Zone gespeist ist, wobei die so gebildete Separatorzone dieser Separatorkette und dieser Destillationszone es ermöglicht, einen Abstrom zu erhalten, der zum größeren Teil Ethylen, einen zum größeren Teil das Buten-1 enthaltenden Abstrom, einen zum größeren Teil Hexen-1 und Octen-1 enthaltenden Abstrom und einen Abstrom umfaßt, der zum größeren Teil die Alpha-Olefine umfaßt, welche wenigstens 10 Kohlenstoffatome pro Molekül, den inhibierten Katalysator und das Lösungsmittel umfaßt.

8. Verfahren nach Anspruch 6, anschließend an die Destillationszone eine Separatorkette umfassend, die wenigstens zum größeren Teil mit dem Bodenprodukt dieser Zone gespeist ist, wobei die so gebildete Zone dieser Separatorkette und dieser Destillationszone es ermöglicht, einen Abstrom zu erhalten, der zum größeren Teil Ethylen umfaßt, einen Abstrom, der zum größeren Teil Buten-1 umfaßt, einen Abstrom, der zum größeren Teil Hexen-1 umfaßt, einen Abstrom, der zum größeren Teil Octen-1 umfaßt und einen Abstrom, der zum größeren Teil die Verbindungen umfaßt, die wenigstens 10 Kohlenstoffatome pro Molekül, den inhibierten Katalysator und das Lösungsmittel umfassen.

9. Verfahren nach Anspruch 6, anschließend an die Destillationszone eine Separatorkette umfassend, die wenigstens zum größeren Teil mit dem Bodenprodukt dieser Zone gespeist ist, wobei die so gebildete Separatorzone dieser Separatorkette und dieser Destillationszone es ermöglicht, einen Abstrom zu erhalten, der zum größeren Teil Ethylen umfaßt, einen Abstrom, der zum größeren Teil Buten-1 umfaßt, einen Abstrom, der zum größeren Teil Hexen-1 umfaßt, einen Abstrom, der zum größeren Teil Octen-1 umfaßt, einen Abstrom, der zum größeren Teil das Lösungsmittel umfaßt und einen Abstrom, der zum größeren Teil die Alpha-Olefine umfaßt, welche wenigstens 10 Kohlenstoffatome pro Molekül sowie den inhibierten Katalysator umfassen.

10. Verfahren nach einem der Ansprüche 6 bis 9, derart, daß ein Teil des Abstroms, der zum größeren Teil das Ethylen umfaßt, zum größeren Teil in die Oligomerisierungszone rezykliert wird.

11. Verfahren nach Anspruch 10, derart, daß dieser Teil zusätzlich zum Ethylen 0 bis 20 Gew. -% Buten-1 umfaßt.
